# EUROPÄISCHE PATENTSCHRIFT

(11) **EP 3 906 119 B1**
(45) Veröffentlichungstag und Bekanntmachung des Hinweises auf die Patenterteilung: **15.10.2025**
(21) Anmeldenummer: 19700633.1
(22) Anmeldetag: 03.01.2019
(51) Int. Cl.: B05B 1/14, B05B 15/40, A61M 11/00, B65D 83/14

(54) **DÜSENEINHEIT, FLÜSSIGKEITSSPENDER MIT EINER SOLCHEN DÜSENEINHEIT UND VERFAHREN ZUR HERSTELLUNG SOLCHER DÜSENEINHEITEN**
NOZZLE UNIT, LIQUID DISPENSER COMPRISING SUCH A NOZZLE UNIT, AND METHOD FOR PRODUCING SUCH NOZZLE UNITS
UNITÉ DE BUSE, DISTRIBUTEUR DE LIQUIDE COMPRENANT UNE TELLE UNITÉ DE BUSE ET PROCÉDÉ POUR LA FABRICATION D'UNE TELLE UNITÉ DE BUSE

(43) Veröffentlichungstag der Anmeldung: 10.11.2021
(73) Patentinhaber: Aptar Radolfzell GmbH, 78315 Radolfzell (DE)
(72) Erfinder: GREINER-PERTH, Jürgen, 78244 Gottmadingen (DE)
(74) Vertreter: Witte, Weller und Partner Patentanwälte mbB Stuttgart
(86) Internationale Anmeldenummer: PCT/EP2019/050115
(87) Internationale Veröffentlichungsnummer: WO 2020/141024

(56) Entgegenhaltungen:
- EP-A1- 2 886 185
- EP-A1- 3 275 558
- EP-B1- 2 890 502
- WO-A1-2015/194962
- WO-A1-2018/097960
- US-A- 3 756 472
- US-A- 4 014 797

## Beschreibung

### ANWENDUNGSGEBIET UND STAND DER TECHNIK

Die Erfindung betrifft ein Verfahren zur Herstellung einer Düseneinheit für einen Flüssigkeitsspender.

Eine Düseneinheit im Sinne der Erfindung ist ein Bauteil eines Flüssigkeitsspenders, welches sich am Ende eines Flüssigkeitspfades befindet, entlang dessen die Flüssigkeit aus einem Flüssigkeitsspeicher des Flüssigkeitsspenders ausgetragen wird. Üblicherweise wird eine solche Düseneinheit zur Erzeugung eines Sprühstrahls verwendet, der dann beispielsweise in ein Mundstück zum Zwecke der Inhalation abgegeben wird.

Die Düseneinheit wird in ein Gehäuse des Flüssigkeitsspenders eingesetzt. Die zur Montage verwendeten Außenflächen der Düseneinheit werden durch einen Kunststoffträger gebildet. Dieser ist von einer Eingangsseite, von der die Flüssigkeit zuströmt, bis zu einer Ausgangsseite, an der die Abgabe erfolgt, von einem Düsenkanal durchdrungen.

Innerhalb des Düsenkanals ist üblicherweise eine Düsenplattenanordnung angeordnet, primär vorliegend eine Düsenplattenanordnung mit einer Vielzahl sehr kleiner Düsenöffnungen, durch die druckbeaufschlagte Flüssigkeit hindurchgedrückt wird. Hierdurch wird die Flüssigkeit in kleine oder kleinste Tröpfchen zerrissen, so dass beispielsweise ein rachen- oder lungengängiges Aerosol erzeugt wird.

Die Düseneinheit dient mit dem Kunststoffträger nicht nur der Anordnung der Düsenplattenanordnung im Strömungspfad, sondern kann auch Träger einer Filteranordnung sein, die ausströmende Flüssigkeit vor der Düsenplattenanordnung von Bestandteilen der Flüssigkeit befreit, die geeignet sein könnten, die Düsenöffnungen zu blockieren und dadurch den bestimmungsgemäßen Austrag zu verhindern.

Eine exemplarische Düseneinheit, die in Hinblick auf den Verwendungszweck der erfindungsgemäßen Düseneinheit ähnelt, ist aus der WO 2015/194962 A1 bekannt. Die dort vorgestellte Düseneinheit verfügt über eine Düsenplatte und verschiedene Filterflächen, die durch thermoplastische Verformung eines Düsenkanals in diesem gehalten sind.

Das Dokument US 3756462 A beschreibt ein Mikroemitter-Gerät für Druckbehälter, das den kontrollierten Ausstoß von Flüssigkeiten ermöglicht. Es verwendet eine perforierte Membran, um Flüssigkeitströpfchen zu zerstäuben.

Das Dokument EP 2890502 B1 beschreibt ein Schaumsprühgerät, das eine Schaumkonsistenz erzeugt. Es verwendet einen zusammendrückbaren Behälter und einen speziellen Mechanismus mit Ventilen, Membranen und Mascheneinsätzen, um Luft und Flüssigkeit zu mischen.

### AUFGABE UND LÖSUNG

Aufgabe der Erfindung ist es, ein zur Herstellung einer Düseneinheit mit hoher Zuverlässigkeit hinsichtlich der Austragcharakteristik geeignetes Herstellungsverfahren zur Verfügung zu stellen.

Das erfindungsgemäße Verfahren dient im Rahmen der Herstellung einer Düseneinheit der Einbringung einer Düsenplattenanordnung in den Dosierkanal.

Die Einbringung der Düsenplattenanordnung erfolgt erfindungsgemäß unter Nutzung eines Montagewerkzeugs. Dieses Montagewerkzeug weist eine Außenkontur mit Übermaß gegenüber dem Düsenkanal auf, so dass das Montagewerkzeug durch Einführung in den Düsenkanal und dortige Kraftbeaufschlagung einer Düsenkanalwandung den Düsenkanal elastisch aufweiten kann. Eine Stirnfläche des Montagewerkzeugs ist dabei größer als eine Außenkontur der Düsenplattenanordnung.

Zur Einbringung der Düsenplattenanordnung wird der Kunststoffträger zunächst in eine definierte Montagelage gebracht, vorzugsweise mit nach unten weisender Ausgangsseite des Kunststoffträgers. Eine solche definierte Montagelage kann absolut ortsfest oder ortsfest an einem als Ganzem beweglichen Montageträger vorgesehen sein.

In der genannten Montagelage wird zunächst die Düsenplattenanordnung von der Eingangsseite aus und in Richtung einer Fügerichtung auf die Ausgangsseite zu in den Düsenkanal eingebracht. Der Düsenkanal weist zu diesem Zweck vorzugsweise einen eingangsseitigen Teilabschnitt auf, dessen Querschnitt größer als die Außenkontur der Düsenplattenanordnung ist, so dass die Düsenplattenanordnung ohne Verformung des Düsenkanals in diesen einlegbar ist.

Die Düsenplattenanordnung kann bei einer besonders einfachen und bevorzugten Gestaltung als einstückige Düsenplatte ausgebildet sein und unmittelbar vor der Einfügung in den Düsenkanal mittels eines Stanzvorgangs aus einer Trägerplatte ausgestanzt worden sein. Hierbei wird unter einer unmittelbare Einfügung verstanden, dass ausgestanzte Düsenplatten nicht zunächst zusammengeführt werden, um in einem späteren Verfahrensschritt in Kunststoffträger eingebracht zu werden. Stattdessen erfolgt gemäß diesem bevorzugten Vorgehen nur eine Einzelhandhabung ausgestanzter Düsenplattenanordnungen. Die Gefahr einer Verschmutzung oder Verletzung der Düsenplattenanordnung ist dadurch sehr gering. Alternativ zu einer nur aus der Düsenplatte bestehenden Düsenplattenordnung kann diese zusätzlich einen Trägerring aufweisen, wie im Weiteren noch erläutert wird.

Nachdem die Düsenplattenanordnung oder im Falle der einfachen Gestaltung die einstückige Düsenplatte in den Düsenkanal eingefügt wurde, wird anschließend das Montagewerkzeug in Fügerichtung von der Eingangsseite in den Düsenkanal eingeführt, gleichsam der Düsenplattenanordnung folgend. Die genannte Reihenfolge kann auch dadurch erzielt werden, dass zunächst die Düsenplatte auf der Stirnseite des Montagewerkzeugs platziert wird und dann das Montagewerkzeug zusammen mit der Düsenplatte in den Düsenkanal eingeschoben wird.

Mit fortschreitendem Einfahren des Montagewerkzeugs in den Düsenkanal in Fügerichtung gelangt das Montagewerkzeug zunehmend in Kontakt mit der Düsenkanalwandung des Düsenkanals und weitet diesen durch seine Außenkontur partiell elastisch auf. Diese Aufweitung gestattet es der Düsenplattenanordnung immer tiefer in den vorzugsweise sich bereits im entspannten Zustand verjüngenden Düsenkanal zu gelangen, ohne dabei im Randbereich verletzt zu werden. Beim zunehmenden Eindrücken kann die Düsenplattenanordnung sich in Berührkontakt mit der Stirnseite des Montagewerkzeugs befinden und von diesem quasi geschoben werden. Ein solcher Berührkontakt ist jedoch nicht zwingend erforderlich. Stattdessen kann die Düsenplattenanordnung beispielsweise alleine durch ihre Gewichtskraft im Zuge des sich aufweitenden Düsenkanals immer tiefer in diesen hineingleiten, während phasenweise oder durchgängig kein Berührkontakt zwischen der Düsenplattenanordnung und der Stirnseite des Montagewerkzeugs besteht. Im Falle einer Düsenplattenanordnung mit Trägerring drückt das Montagewerkzeug vorzugsweise unmittelbar gegen diesen Trägerring.

Wenn die Relativbewegung zwischen dem Kunststoffträger und dem Montagewerkzeug endet, hat die Düsenplattenanordnung ihre Endlage erreicht.

Das Montagewerkzeug wird nun entgegen der Fügerichtung aus dem Düsenkanal herausgezogen. Die Düsenplattenanordnung verbleibt im Wesentlichen in ihrer Endlage, wobei sich diese noch etwas verändern kann, wenn die Düsenplattenanordnung unter dem Eindruck der Rückstellung des Düsenkanals ebenfalls ein kleines Stück weit entgegen der Fügerichtung zurückgeschoben wird. Die Rückstellung des Düsenkanals beim Rückzug des Montagewerkzeugs bewirkt, dass schlussendlich die Düsenplattenanordnung durch die Düsenkanalwandung gehalten ist, insbesondere durch zwei Düsenkanalabschnitte beidseitig der Düsenplatte, deren nach Rückstellung verbleibender lichter Querschnitt kleiner als die Außenkontur der Düsenplattenanordnung ist.

Im Bereich der Düsenplattenanordnung drückt sich diese von innen gegen die sich rückstellende Wandung des Düsenkanals und verhindert somit dessen vollständige Rückstellung. Die Düsenplattenanordnung ist somit im Düsenkanal gleichsam eingeklemmt. Stromabwärts und stromaufwärts der Düsenplattenanordnung schnürt die rückstellende Wandung den Düsenkanal vorzugsweise leicht ein, so dass die Düsenplattenanordnung nach Entfernung des Montagewerkzeugs formschlüssig gehalten ist.

Wenngleich das Verfahren grundsätzlich auch zum Einsetzen von Düsenplattenanordnungen in zylindrische Düsenkanäle genutzt werden kann, ist es von Vorteil, wenn sich der Düsenkanal in Fügerichtung, also von der Eingangsseite zur Ausgangsseite, verjüngt. Insbesondere vorzugsweise weist der Düsenkanal mindestens einen konischen Teilabschnitt auf, wobei dieser vorzugsweise an seinem in Richtung der Eingangsseite weisenden Ende einen Querschnitt aufweist, der größer als die Außenkontur des Montagewerkzeugs ist, und der an seinem in Richtung der Ausgangsseite weisenden Ende einen Querschnitt aufweist, der kleiner als die Außenkontur des Montagewerkzeugs ist.

Bei der Einführung des Montagewerkzeugs in den Düsenkanal gelangt das Montagewerkzeug mit seiner Stirnfläche und/oder Außenkontur im genannten konischen Teilabschnitt in Kontakt mit der Düsenkanalwandung des Düsenkanals und weitet diese bei fortgesetzter Bewegung. Die konische Formgebung führt zu einem gut reproduzierbaren Einrücken der Düsenplattenanordnung in einen durch das Montagewerkzeug stetig fortschreitendend sich aufweitenden Bereich des Düsenkanals. Ein bevorzugter Öffnungswinkel des konischen Teilabschnitts liegt im Bereich zwischen 5° und 15°.

Während des fortschreitenden Einfahrens des Montagewerkzeugs in den Düsenkanal in Fügerichtung kann von der Ausgangseite aus ein Hilfswerkzeug fluchtend mit Montagewerkzeug in den Düsenkanal eingeschoben sein. Dieses kann insbesondere in Fügerichtung herausgezogen werden, während das Montagewerkzeug noch ebenfalls in Fügerichtung in den Düsenkanal einrückt.

Ja nach Ausgestaltung und Bewegungsprofil kann das Hilfswerkzeug verhindern, dass die Düsenplatte unbeabsichtigt zu weit in den Düsenkanal einrückt. Es kann darüber hinaus auch helfen, eine genaue Position der Endlage und/oder eine bestimmungsgemäße Ausrichtung einzuhalten.

Bei einer besonderen Ausgestaltung des Verfahrens liegen die Stirnfläche des Montagewerkzeugs und/oder eine Stirnfläche des Hilfswerkzeugs während des fortgesetzten Einrückens der Düsenplattenanordnung in den Düsenkanal an dieser an. Liegen beide Stirnflächen an, so kann die Position der Düsenplattenanordnung im Düsenkanal genau gesteuert werden.

Zudem kann durch die Gestaltung der Stirnflächen des Montagewerkzeugs und/oder des Hilfswerkzeugs mit nichtebener Form eine Verformung der Düsenplattenanordnung bewirkt werden. Insbesondere kann die Stirnfläche des Montagewerkzeugs eine konvex gewölbte Formgebung aufweisen und die Stirnfläche des Hilfswerkzeugs eine konkav gewölbte Stirnfläche aufweisen. Während des Einfahrens des Montagewerkzeugs in den Düsenkanal drücken die derart geformten Stirnflächen die Düsenplattenanordnung elastisch und/oder plastisch in eine gewölbte Form.

Eine solche gewölbte Form bietet den Vorteil, dass die zuvor parallelen Düsenöffnungen gegeneinander angewinkelt werden und insbesondere divergierend ausgerichtet werden. Dies ist bei vielen Anwendungsfällen von Vorteil, da es einen breiter aufgefächerten Sprühstrahl bewirkt.

Zudem führt eine durch das Montagewerkzeug und/oder das Hilfswerkzeug und insbesondere durch die beiden Werkzeuge gemeinsam bewirkte elastische Verformung der Düsenplatte dazu, dass der Durchmesser der Düsenplattenanordnung temporär verkleinert wird so dass die Düsenplattenanordnung im Falle eines sich verjüngenden Düsenkanals tiefer in diesen einrücken kann. Sobald die Kraftbeaufschlagung der Düsenplatte durch das Montagewerkzeug und/oder das Hilfswerkzeug entfällt, stellt sich daher eine besonders starke Verformung eines umgebenden Ringbereichs ein und damit eine hohe Lagestabilität der Düsenplattenanordnung.

Das erfindungsgemäße Verfahren führt aufgrund der üblicherweise eintretenden elastischen Verformung eines die Düsenplattenanordnung umgebenden Ringbereichs sowie der Bauweise der einzelnen Komponenten, die auch im weiteren noch näher erläutert sind, ohne thermische Nachbehandlung zu einer hohen Dichtigkeit und Lagestabilität der Düsenplattenanordnung. Dennoch kann es von Vorteil sein, wenn die Düsenplattenanordnung nach Erreichen ihrer Endlage und nach Entfernen des Montagewerkzeugs bis zur Erweichungstemperatur des Kunststoffs des Kunststoffträgers und insbesondere auf mindestens 100°C erhitzt wird, so dass die Dichtigkeit im Randbereich der Düsenplattenanordnung nochmals verbessert wird. Diese Erwärmung findet jedoch vorzugsweise nicht in einem Maße statt, welches einen vollständigen Abbau der elastischen Deformation im genannten Ringbereich zur Folge hätte. Die Erwärmung kann beispielsweise mittels eines Lasers oder per Induktion bewirkt werden.

Eine durch das Verfahren hergestellte Düseneinheit verfügt über einen Kunststoffträger, der von einer Eingangsseite bis zu einer Ausgangsseite von einem Düsenkanal durchdrungen ist. Der Kunststoffträger weist vorzugsweise eine zum Düsenkanal koaxial rotationssymmetrische Außenform auf, um während der Montage in einen Flüssigkeitsspender einfach handhabbar zu sein.

In dem Düsenkanal der Düseneinheit ist eine Düsenplattenanordnung angeordnet. Diese weist eine Vielzahl von Düsenöffnungen auf, vorzugweise mindestens 10, insbesondere vorzugsweise mindestens 30. Der mittlere Durchmesser dieser Düsenöffnungen liegt vorzugsweise zwischen 1 µm und 100 µm auf, insbesondere vorzugsweise zwischen 2 µm und 10 µm. Diese Durchmesserangaben beziehen sich im Falle nichtrunder Düsenöffnungen auf gedachte runde Düsenöffnungen mit zu den nichtrunden Düsenöffnungen gleichgroßem lichten Querschnitt.

Die Düsenplattenanordnung ist unter elastischer Verformung des Kunststoffträgers in diesen eingefügt, so dass zumindest ein die Düsenplattenanordnung umgebender Ringbereich des Kunststoffträgers sich in einem elastisch komprimierten Zustand befindet und hierdurch eine sichere und dichte Befestigung der Düsenplattenanordnung ermöglicht.

Die Düsenplattenanordnung ist bei einer bevorzugten Gestaltung einstückig ausgebildet, insbesondere in Form einer flachen Düsenplatte mit im Wesentlichen konstanter Dicke. In dieser Düsenplatte sind die Düsenöffnungen vorgesehen. Die Außenkontur dieser Düsenplatte wirkt auf den komprimierten Ringbereich des Kunststoffträgers und deformiert diesen hierdurch. Vorzugsweise weist die Düsenplatte einen Randbereich auf, in welchem die Dicke der Düsenplatte verjüngt, um mit einem besonders schmalen Grat auf den Ringbereich zu wirken und hierdurch in die innere Oberfläche des Ringbereichs einzudringen. Hierdurch ist ein sehr guter Halt gewährleistet. Trotz des Eindringens verbleibt eine elastische Komprimierung des Ringbereichs, die langfristig die Dichtigkeit sichert und somit einer Verschlechterung des Sprühbildes oder einem Eindringen von Verschmutzungen auch bei langen Lagerzeiten entgegenwirkt.

Neben der beschriebenen einstückigen Gestaltung können je nach Anwendungszweck und Art der Montage auch andere Gestaltungen der Düsenplattenanordnung zweckmäßig sein. Hierzu gehört insbesondere eine Gestaltung der Düsenplattenanordnung mit einer flachen Düsenplatte und zusätzlich einem hieran angebrachten Trägerrahmen. Der Trägerrahmen weist eine zentrische Durchbrechung auf, die die Düsenöffnungen der Düsenplatte frei lässt. Der Trägerrahmen kann selbst jener Teil der Düsenplattenanordnung sein, der den Ringbereich nach außen radial kraftbeaufschlagt. Vorzugsweise jedoch ist es auch bei Vorhandensein eines Trägerrahmens ausschließlich oder zumindest überwiegend die Düsenplatte, die mit ihrer vorzugsweise dünnwandigen Außenkontur unmittelbar auf den Ringbereich wirkt. Der Trägerrahmen weist daher in diesem Falle eine Außenkontur auf, die ausreichend klein ist, dass die Außenkontur der Düsenplatte den Ringbereich elastisch verformen kann.

Der Trägerrahmen kann die Handhabung von Düsenplattenanordnungen im Vorfeld der Montage sowie die Montage selbst erleichtern. Er kann durch Anbringung in stromaufwärtiger Richtung an der Düsenplatte insbesondere dem Zweck dienen, die durch das beschriebene Montagewerkzeug direkt in die Düsenplatte eingekoppelte Kraft aufzunehmen.

Sofern ein Trägerrahmen vorgesehen ist, ist dieser vorzugsweise aus Kunststoff hergestellt. Die Düsenplatte ist vorzugsweise aus einem metallischen Werkstoff, vorzugsweise aus Nickel, hergestellt. Als Fertigungsverfahren kommt insbesondere das additive Herstellungsverfahren des "Electroforming" in Frage. Hierbei können diese Düsenöffnungen bereits in einem einstufigen Verfahren erzeugt werden. Alternativ können Düsenöffnungen auch per Laser nachträglich erzeugt werden. Neben metallischen Düsenplatten sind auch Düsenplatten aus anderen Materialien verwendbar, beispielsweise Silizium-Düsenplatten.

Die Düsenplatte weist im einfachsten Falle eine ausreichende Eigenstabilität auf, um auch unter dem Eindruck der durch den Ringbereich erzeugten Kraftwirkung eben zu bleiben. Von Vorteil kann jedoch auch eine Gestaltung sein, bei der die Düsenplatte eine gewölbte Formgebung aufweist. Diese gewölbte Formgebung kann durch plastische Deformation, insbesondere im Zuge des oben beschriebenen Montageverfahrens oder bereits vor der Montage, erzeugt werden. Die gewölbte Formgebung kann jedoch je nach Art des verwendeten Materials der Düsenplatte auch durch elastische Verformung und einen somit verbleibenden Spannungszustand hergestellt sein.

Der Düsenkanal weist vorzugsweise eine rotationssymmetrische Form auf. Auch die Düsenplattenanordnung weist vorzugsweise eine runde Außenkontur auf, da dies die Montage vereinfacht und eine dichte Verbindung mit dem Ringbereich begünstigt.

An seinem Richtung Eingangsseite weisenden Ende weist der Düsenkanal vorzugsweise einen lichten Querschnitt auf, der größer ist als die Außenkontur der Düsenplattenanordnung. Dadurch ist die Montage erheblich erleichtert. Zudem ist der große Querschnitt oder eine den Düsenkanal umgebende Vertiefung für das Vorsehen eines stromaufwärtigen Filters von Vorteil, wie im Weiteren noch erläutert wird.

Der Düsenkanal weist zum Zwecke der einfachen Montage vorzugsweise eine sich verjüngende Formgebung auf. Insbesondere vorzugsweise weist der Düsenkanal mindestens einen konischen Teilabschnitt mit einem Öffnungswinkel zwischen 5° und 15° auf. Ein konischer Teilabschnitt dieser Steigung eignet sich sehr gut für die Montage mittels oben beschriebenen Montagewerkzeug.

Vorzugsweise verfügt der Düsenkanal über eine Mehrzahl von Teilabschnitten, die jeweils konisch geformt sind, jedoch unterschiedliche Öffnungswinkel aufweisen. So ist vor allem neben dem oben genannten Teilabschnitt mit einem Öffnungswinkel zwischen 5° und 15° ein weiterer Teilabschnitt mit einem größeren Öffnungswinkel. In diesem Bereich befindet sich vorzugsweise die Endlage der Düsenplattenanordnung. Der größere Öffnungswinkel verbessert Genauigkeit der Einhaltung einer bestimmungsgemäßen Endlage der Düsenplattenanordnung, also ihrer Einbaulage, sowie die Reproduzierbarkeit dieser Endlage in der Massenherstellung.

Stromabwärts der Düsenplattenanordnung verjüngt sich der Düsenkanal vorzugsweise weiter, so dass auf die Düsenplattenanordnung ein nach innen weisender Haltebereich des Düsenkanals folgt, dessen lichter Querschnittsdurchmesser mindestens 10% kleiner als der Durchmesser der Düsenplattenanordnung ist. In einer bevorzugten Ausgestaltung ist die Außenkontur der Düsenplattenanordnung umlaufend durch einen in diesem Maße gegenüber der Außenkontur verjüngten Haltebereich gehalten. Der Haltebereich sichert die Düsenplattenordnung, so dass während der Montage oder Fertigungshandhabung sowie im Gebrauch gewährleistet ist, dass selbst bei besonderer mechanischer Belastung wie Flüssigkeitsdruckspitzen die Düsenplattenanordnung nicht in Strömungsrichtung aus dem Düsenkanal herausgedrückt werden kann.

Um eine in Hinblick auf Stabilität und Dichtigkeit im Randbereich besonders vorteilhafte Verbindung zwischen Düsenplattenanordnung und Kunststoffträger zu erzielen, ist der Kunststoffträger in dem die Düsenplatte in ihrer Endlage umgebenden deformierten Ringbereich durch eine rohrartig den Düsenkanal umgebende Wandung gebildet, deren Wandungsstärke im nicht deformierten Zustand zumindest abschnittsweise zwischen 10% und 80% der lichten Breite des Düsenkanals an der Düsenplatte beträgt, vorzugsweise zwischen 20% und 40%. Der Außendurchmesser der rohrförmigen Düsenkanalwandung liegt vorzugsweise zwischen 3 mm und 15 mm. Die Wandungsstärke liegt vorzugsweise zwischen 10% und 30% dieses Außendurchmessers.

Der genannte Kunststoffträger einer erfindungsgemäßen Düseneinheit, welches eine Düsenplattenanordnung und/oder einen Filter der beschriebenen Art aufweist, ist insbesondere vorzugsweise aus PET hergestellt.

Eine Düseneinheit der beschriebenen Art findet bestimmungsgemäß in einem Flüssigkeitsspender Verwendung. Ein solcher Flüssigkeitsspender kann vor allem für den Austrag kosmetischer oder pharmazeutischer Flüssigkeiten ausgebildet sein. Im Falle pharmazeutischer Flüssigkeiten kann es sich insbesondere um solche handelt, die inhaliert werden und zu diesem Zweck mittels der Düseneinheit vernebelt werden.

Er weist üblicherweise einen Flüssigkeitsspeicher sowie ein Gehäuse auf, in das die Düseneinheit der beschriebenen Art eingesetzt ist, um die aus dem Flüssigspeicher geförderte Flüssigkeit durch die Düseneinheit hindurch auszutragen.

Der Flüssigkeitsspender ist dabei vorzugsweise ein kleiner tragbarer Flüssigkeitsspender mit einem Flüssigkeitsspeicher, dessen Maximalvolumen zwischen 10 ml und 1000 ml liegt, vorzugsweise zwischen 50 ml und 250 ml.

Die Förderung vom Flüssigkeitsspeicher zur Düseneinheit kann beispielsweise über eine vorzugsweise manuell betätigbare Pumpeinrichtung erfolgen. Alternativ kann der Flüssigkeitsspeicher als Druckspeicher ausgebildet sein, in welchem die Flüssigkeit unter Druck gelagert ist.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 zeigt einen erfindungsgemäßen Flüssigkeitsspender, der exemplarisch eine übliche Verwendung einer erfindungsgemäßen Düseneinheit verdeutlicht.
Fig. 2 bis 4 zeigen in einer Explosionsdarstellung, einer Einzelteildarstellung sowie einer Schnittdarstellung ein erstes Ausführungsbeispiel einer erfindungsgemäßen Düseneinheit und von deren Einzelteilen.
Fig. 4A bis 4D zeigen Details und Detailvarianten zu Fig. 4.
Fig. 5A bis 5G zeigen ein erstes Teilverfahren zur Herstellung der Düseneinheit der Fig. 2 bis 4, im Rahmen dessen eine Düsenplattenanordnung in den Düsenkanal der Düseneinheit eingefügt wird.
Fig. 6A bis 6C verdeutlichen ein zweites Teilverfahren zur Herstellung der Düseneinheit der Fig. 2 bis 4, im Rahmen dessen ein Filter an der Düseneinheit abgebracht wird.
Fig. 7 und 8 zeigen ein zweites Ausführungsbeispiel einer erfindungsgemäßen Düseneinheit in einer Schnittdarstellung und einer Explosionsdarstellung.
Fig. 9 bis 11 zeigen weitere Ausführungsbeispiele einer erfindungsgemäßen Düseneinheit, die zur Befestigung des Filters über ein zusätzliches Klemmelement verfügen.
Fig. 12 und 13 zeigen ein Ausführungsbeispiel der Düseneinheit mit gewölbter Düsenplatte sowie das Verfahren zur Einbringung dieser Düsenplatte in den Düsenkanal.

### DETAILLIERTE BESCHREIBUNG DER AUSFÜHRUNGSBEISPIELE

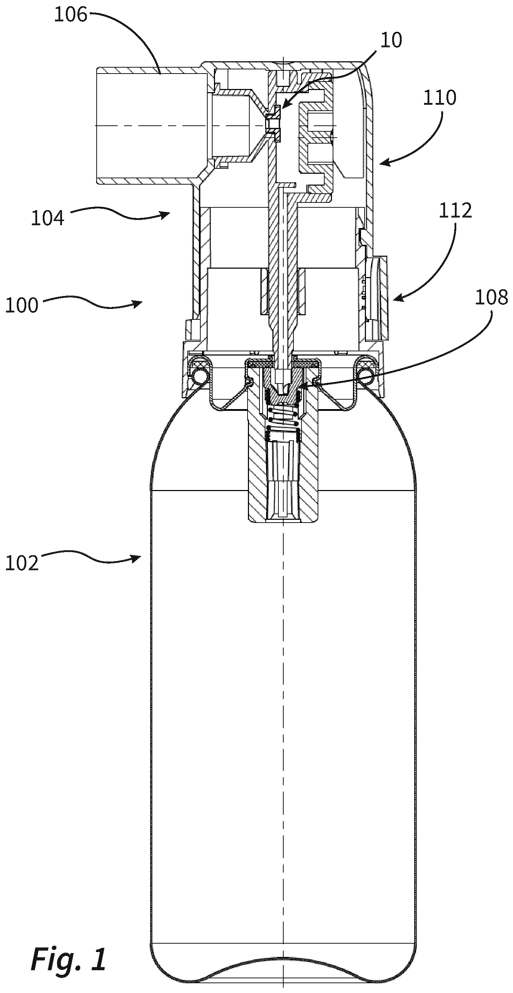

Fig. 1 zeigt einen Flüssigkeitsspender 100 mit einer erfindungsgemäßen Düseneinheit 10. Dieser Flüssigkeitsspender 100 ist als exemplarischer Flüssigkeitsspender für eine Düseneinheit 10 zu verstehen. Es sind auch viele anderweitige Bauformen von Flüssigkeitsspendern unter Nutzung von erfindungsgemäßen Düseneinheiten 10 denkbar.

Der Flüssigkeitsspender 100 der Fig. 1 verfügt über einen Druckspeicher 102, in dem Flüssigkeit vor dem Austrag gelagert ist. An diesem Druckspeicher 102 ist ein Austragkopf mit einem Gehäuse 104 angebracht. Dieser Austragkopf verfügt über eine Basis 112 sowie einen demgegenüber niederdrückbaren Betätigungsdrücker 110. Wenn dieser Betätigungsdrücker 110 niedergedrückt wird, wirkt er auf ein Auslassventil 108 des Druckspeichers 102, so dass Flüssigkeit in den Austragkopf strömt und zur Düseneinheit 10 gelangt. Stromabwärts der Düseneinheit 10 ist ein Auslassstück 106 vorgesehen, vorliegend exemplarisch in Art eines Mundstücks 106. Mittels der Düseneinheit 10 wird die zuströmende Flüssigkeit in die Form eines Sprühstrahls gebracht, der in das Auslassstück 106 abgegeben wird und von einem Benutzer inhaliert werden kann.

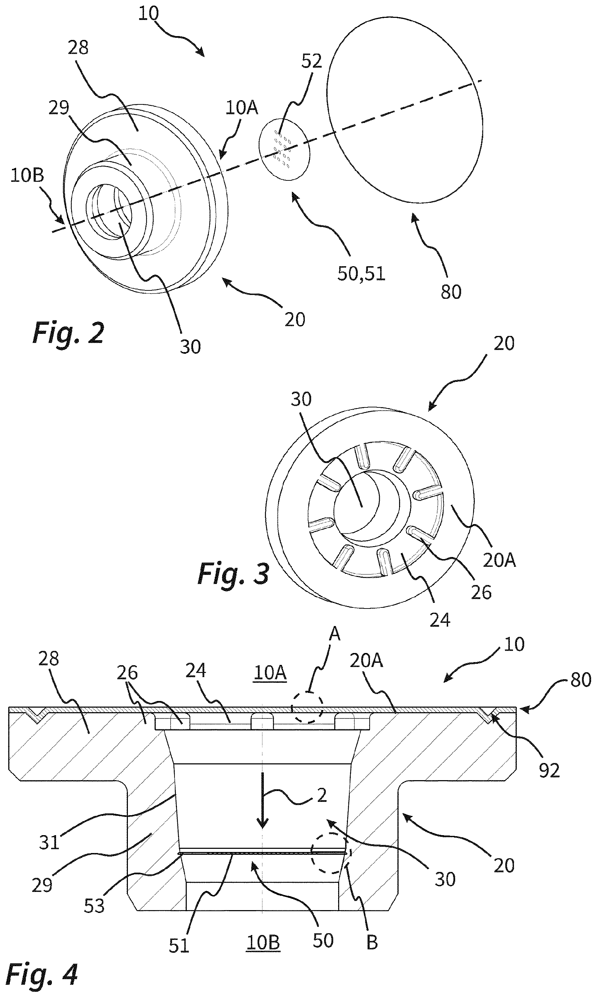

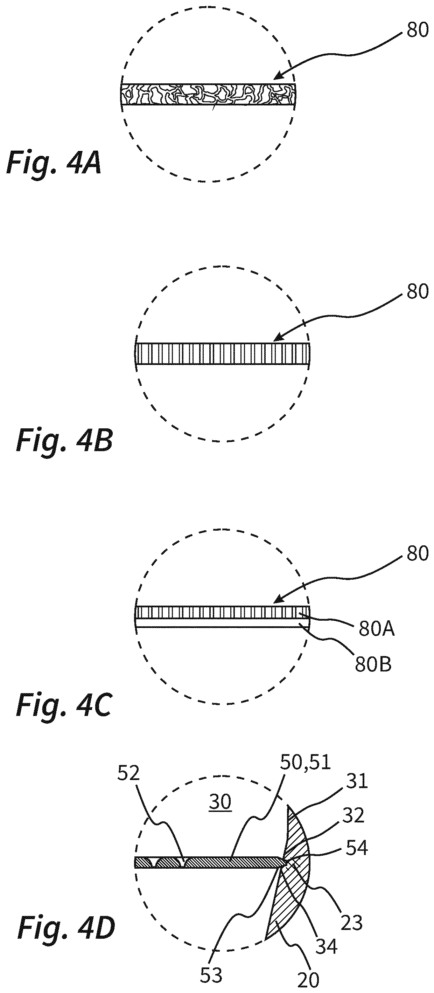

Die Fig. 2 bis 4 sowie 4A bis 4D verdeutlichen zunächst an einem ersten Ausführungsbeispiel mitsamt Varianten den Aufbau einer erfindungsgemäßen Düseneinheit 10.

Fig. 2 zeigt die Einzelkomponenten in einer Explosionsdarstellung.

Die Düseneinheit 10 verfügt als tragende Komponente über einen Kunststoffträger 20, der in etwa die Form eines Zylinderhuts mit einem Krempenabschnitt 28 sowie einem Zylinderabschnitt 29 aufweist. Der Kunststoffträger ist von einer Eingangsseite 10A zu einer Ausgangsseite 10B von einem Düsenkanal 30 durchdrungen.

Von einer Eingangsseite 10A aus eingefügt bzw. angefügt sind eine Düsenplattenanordnung 50 und ein Filter 80. Beim Ausführungsbeispiel der Fig. 2 bis 4 ist die Düsenplattenanordnung 50 alleine durch eine dünne Düsenplatte 51 gebildet wird. In dieser Düsenplatte 51 ist eine Vielzahl von Düsenöffnungen 52 vorgesehen, die in einer matrixartigen Anordnung vorgesehen sind. Der Filter 80 ist in im Weiteren noch näher erläuterter Art und Weise auf einer in Richtung der Eingangsseite 10A weisende Stirnfläche 20A des Kunststoffträgers 20 aufgebracht.

Fig. 3 zeigt den Kunststoffträger 20 von der gegenüberliegenden Seite, also die Eingangsseite 10A des Kunststoffträgers 20. Hier ist zu ersehen, dass innenseitig der umlaufenden Stirnfläche 20A eine Vertiefung 24 den Düsenkanal 30 umgebend vorgesehen ist, in die von außen Stützrippen 26 hineinragen.

Bezug nehmend auf Fig. 4 ist der Aufbau der Düseneinheit 10 im montierten Zustand zu sehen. Es ist zu erkennen, dass die Düsenplatte 51 mit ihrem Randbereich 53 in eine Düsenkanalwandung 31 eingedrungen und hierdurch fixiert ist. Weiter ist zu ersehen, dass der Düsenkanal 30 eine als Ganzes sich verjüngende Formgebung in Richtung der Fügerichtung 2 aufweist, wobei verschiedene konische Teilabschnitte vorgesehen sind.

Der Filter 80 ist auf der Stirnfläche 20A positioniert und im Bereich einer umlaufenden Schweißstelle 92 mit dem Kunststoffträger 20 verschweißt. Durch die Vertiefung 24 ist die wirksame Fläche des Filters 80 sehr groß, vorliegend etwa doppelt so groß wie Querschnitt des Düsenkanals 30 an seiner engsten Stelle. Der Filter 80 kann hierdurch vergleichsweise große Flüssigkeitsmengen filtern ohne zu verstopfen.

Der Filter 80 kann exemplarisch eine Trenngrenze von 4 µm aufweisen, also alle oder nahezu alle Partikel herausfiltern, die bei Poren entsprechender Größe den Filter nicht passieren können. Die genannte Trenngrenze von 4 µm ist gut geeignet, wenn die Düsenöffnungen 52 einen lichten Querschnitt von 8 µm aufweisen. Durch diese Abstimmung ist gewährleistet, dass alle Bestandteile der Flüssigkeit, die den Filter 80 durchqueren können, auch durch die Düsenöffnungen hindurch abgegeben werden können.

Wie die Fig. 4A bis 4C verdeutlichen, kann der Filter 80 in verschiedener Art und Weise ausgestaltet sein. Die Fig. 4A bis 4C zeigen Varianten des in Fig. 4 gekennzeichneten Bereichs A.

Bei der Gestaltung gemäß Fig. 4A findet ein Tiefenfilter Verwendung, also ein Filter 80 aus einem porösen und beispielsweise gesinterten Material, welches von unregelmäßigen Poren durchdrungen ist, die im Ergebnis dazu führen, dass Partikel bestimmter Größe den Tiefenfilter nicht durchdringen können, sondern in diesem festgehalten werden.

Fig. 4B zeigt eine Gestaltung mit einem Membranfilter als Filter 80. Dieser weist Filteröffnungen 82 auf, die eine definierte Position und Form haben und beispielsweise mittels eines Laserstrahls in den Filter 80 bzw. dessen Filtermaterial 180 eingebracht worden sein können. Partikel, die größer als der Querschnitt dieser Filteröffnungen sind, können den Filter 80 nicht durchdringen und sammeln sich auf der stromaufwärtigen Seite des Filters 80.

In Fig. 4C ist eine Variante dargestellt, bei der ebenfalls ein Membranfilter als Filter 80 Verwendung findet. Dieser verfügt über eine Filtermembran 80A ähnlich jener der Fig. 4B. Zusätzlich ist eine Trägerschicht 80B aus einem groben Vlies vorgesehen, die der Filtermembran 80A die notwendige Stabilität gibt.

Die Fig. 4D zeigt den Bereich B der Fig. 4, in dem die Düsenplatte 51 an der Düsenkanalwandung 31 anliegt. Es ist zu ersehen, dass die von Düsenöffnungen 52 durchdrungene Düsenplatte 51 im Randbereich 53 eine sich verjüngende Formgebung aufweist und dass die Außenkontur 54 der Düsenplatte 51 aufgrund des im Folgenden noch beschriebenen Montageverfahrens dazu führt, dass im Bereich der Düsenkanalwandung 31 eine Kompressionszone 23 entsteht, in der das Kunststoffmaterial des Kunststoffträgers 20 komprimiert ist. Stromabwärts und stromaufwärts der Düsenplatte 51 ragt die Düsenkanalwandung 31 über den Randbereich 53 der Düsenplatte 51 nach innen hinaus, so dass die Düsenplatte 51 formschlüssig gesichert ist.

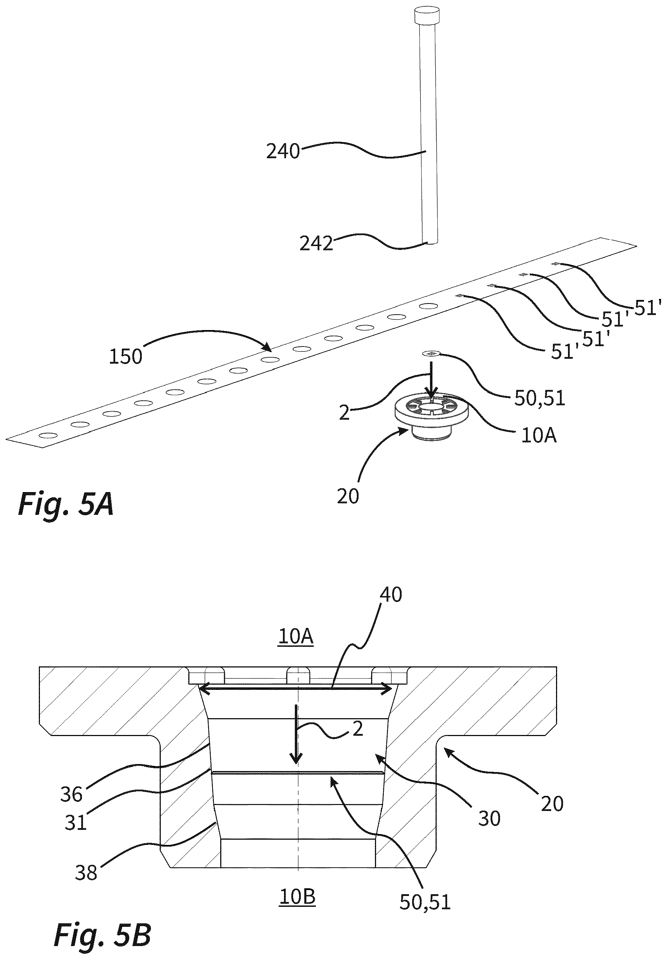

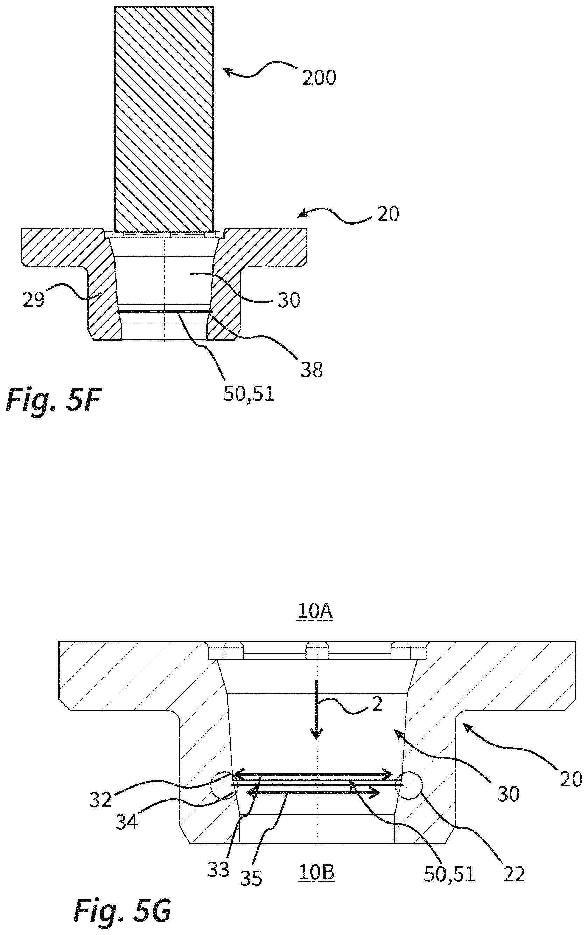

Die Fig. 5A bis 5G zeigen das Verfahren zur Einbringung der Düsenplatte 51 in den Kunststoffträger 20.

Wie in Fig. 5A dargestellt, wird zunächst ausgehend von einer Trägerplatte 150 mit einer Vielzahl von Düsenplattenbereichen 51' mit Düsenöffnungen 52 mittels eines Stanzwerkzeugs und dessen stirnseitiger Stanzfläche 242 eine Düsenplatte 51 ausgestanzt, welche bei der vorliegenden Gestaltung alleine die Düsenplattenanordnung 50 bildet. Diese Düsenplatte 51 wird unmittelbar nach dem Stanzen, also ohne zwischenzeitliche gemeinsame Lagerung mit anderen Düsenplatten, in Fügerichtung 2 von der Eingangsseite 10A aus in den Kunststoffträger 20 eingesetzt.

Wie in Fig. 5B ersichtlich ist, gelangt die Düsenplatte 51 aufgrund eines Querschnitts 40 an der Eingangsseite, der größer als die Außenkontur 54 der Düsenplatte 51 ist, unmittelbar recht tief in den Düsenkanal 30 hinein und kommt zunächst in einem konischen Teilabschnitt 36 der Düsenkanalwandung 31 zum Anliegen.

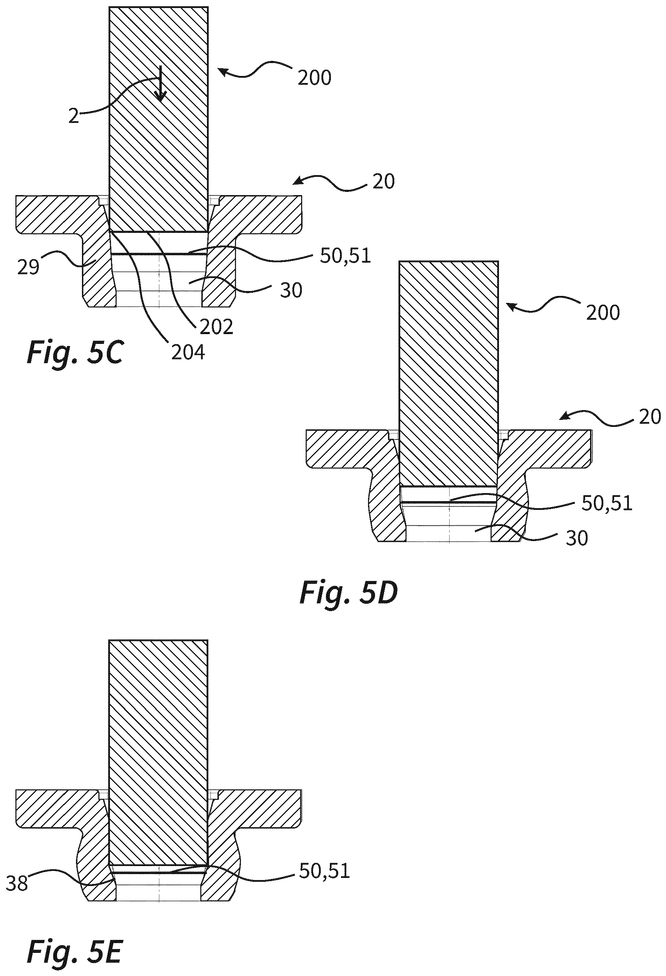

Wie in Fig. 5C verdeutlicht ist, wird anschließend ein Montagewerkzeug 200 von oben in Fügerichtung 2 in den Düsenkanal 30 eingeschoben. Mit seiner Außenkontur 202 kommt das Montagewerkzeug 200 ebenfalls im konischen Teilabschnitt 36 in Kontakt mit der Düsenkanalwandung 31. Im Zuge der fortgesetzten Bewegung des Montagewerkzeugs 200 in Fügerichtung 2 beginnt dieses, wie in Fig. 5D ersichtlich ist, den Düsenkanal 30 elastisch aufzuweiten.

Durch dieses Aufweiten sinkt auch die Düsenplatte 51 zunehmend in Fügerichtung nach unten, bis sie in einem zweiten konischen Teilabschnitt 38 ihre in Fig. 5E dargestellte Endlage erreicht. Beim vorliegenden Beispiel bedarf es für dieses Weiterbewegen der Düsenplatte 51 keines direkten Kontaktes mit dem Montagewerkzeug 200. Bei anderen Ausgestaltungen des Verfahrens kann jedoch auch vorgesehen sein, dass das Montagewerkzeug 200 in Kontakt mit der Düsenplatte 51 ist und diese dadurch tiefer in den Düsenkanal 30 einschieben kann.

Abschließend wird in der in Fig. 5F verdeutlichten Weise das Montagewerkzeug 200 entgegen der Fügerichtung 2 aus dem Düsenkanal 30 herausgezogen. Die Düsenplatte 51 verbleibt im Düsenkanal 30. Die Düsenkanalwandung 31, die zwischenzeitlich elastisch aufgeweitet war, kehrt in ihre Ausgangslage zurück, wobei sie in einem Ringbereich 22 im Bereich der Endlage der Düsenplatte 51 aufgrund der Düsenplatte 51 sich nicht vollständig zurückstellen kann, so dass in einem umlaufenden Ringbereich 22 die bereits genannte Kompressionszone 23 verbleibt, die in Fig. 4D dargestellt ist. Beidseitig dieser Kompressionszone 23 im Bereich der Düsenkanalabschnitte 32, 34 erfolgt jedoch eine Rückstellung der Düsenkanalwandung 31. Die Düsenkanalwandung 31 kehrt dabei so weit in ihre Ausgangslage zurück, dass die dortigen lichten Querschnitte 33, 35 kleiner sind als die Außenkontur 54 der Düsenplatte 51.

Das beschrieben Montageverfahren führt zu einer sicheren Befestigung der Düsenplatte 51 im Düsenkanal 30. Auch externe Kraftbeaufschlagungen im Zuge der Montage und Druckspitzen im Betrieb können die Düsenplatte 51 nicht lösen. Die verbleibende elastische Komprimierung in der Kompressionszone 23 sorgt dafür, dass die Düsenplatte 51 auch im Falle langer Lagerzeiten sicher gehalten ist.

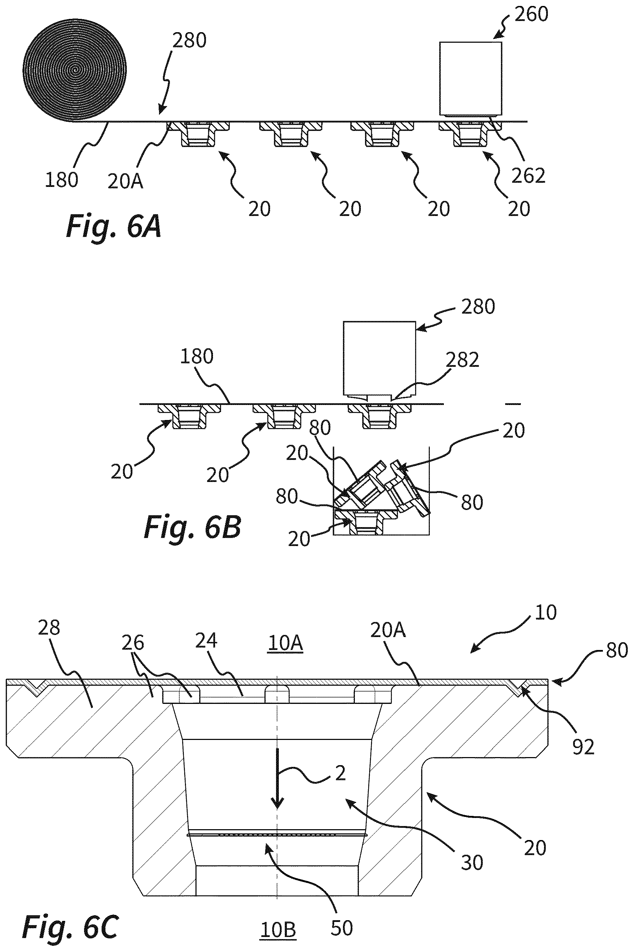

Die Fig. 6A bis 6C verdeutlichen die Aufbringung des Filters 80, wobei das Verfahren vorzugsweise mit noch nicht fertiggestellten Düseneinheiten 10 durchgeführt wird, die entsprechend der Beschreibung der Fig. 5A bis 5G montiert wurden.

Bezug nehmend auf Fig. 6A ist zu ersehen, dass ein Filtermaterial 180 Verwendung findet, welches beispielsweise von einer Rolle abgewickelt werden kann. Dieses wird über die bereits mit der Düsenplattenanordnung 50 versehenen Kunststoffträger 20 gelegt, so dass, vorzugsweise im Rahmen eines kontinuierlichen Prozesses, anschließend mittels eines Fügestempels 260 und einer daran vorgesehenen umlaufenden Fügekante 262 die Kunststoffträger 20 an das Filtermaterial 180 angeschweißt werden können.

Es ergibt sich somit ein Abschnitt des Filtermaterials 180, an dem eine Vielzahl von Kunststoffträgern 20 mit Düsenplattenanordnungen 50 thermisch befestigt ist. Ausgehend hiervon wird in der durch Fig. 6B verdeutlichten Weise mittels eines Schneidwerkzeugs 280 das Filtermaterial 180 umlaufend um die Stirnfläche 20A geschnitten. Die hierdurch fertiggestellten, jedoch noch nicht montierten Düseneinheiten 10 können in der in Fig. 6B angedeuteten Weise unproblematisch gehandhabt werden. Durch den bereits aufgebrachten Filter 80 und den aufgrund des beschriebenen Herstellungsverfahrens von störenden Partikeln freien Innenraum der Düseneinheiten 10 sowie aufgrund des sicheren Verschluss der Düseneinheiten 10 durch die Düsenplatten 51 besteht keinerlei Gefahr, dass eine im Betrieb störende Verschmutzung der so fertiggestellten Düseneinheiten 10 zu befürchten ist.

Fig. 6C zeigt nochmals die fertigen Düseneinheiten 10 mit den nach dem Schneideprozess verbleibenden Filtern 80, die im Bereich der umlaufenden Schweißstelle 92 durch die Fügekante 262 dicht verschlossen sind.

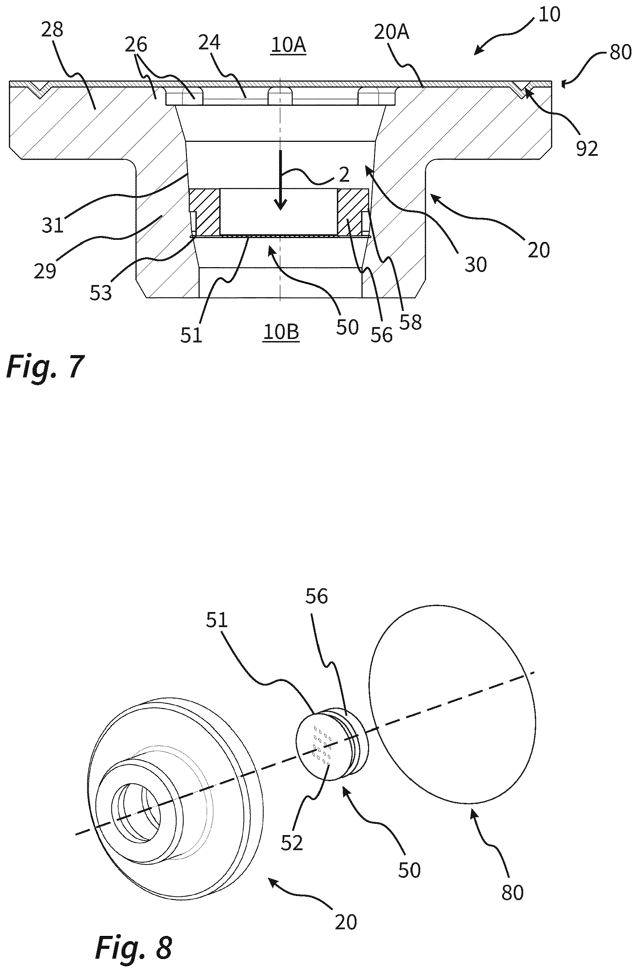

Die Fig. 7 und 8 zeigen eine alternative Gestaltung der Düseneinheit 10. Wesentlicher Unterschied gegenüber der Düseneinheit 10 der Fig. 4 ist die Ausgestaltung der Düsenplattenanordnung 50. Diese besteht vorliegend nicht nur aus der Düsenplatte 51, sondern umfasst zusätzlich einen Trägerrahmen 56 aus Kunststoff, der auf der stromaufwärtigen Seite der Düsenplatte 51 vorgesehen ist. Der Trägerrahmen 56 kann an die Düsenplatte beispielsweise angespritzt sein. Er weist eine Formgebung und insbesondere eine Außenkontur 58 auf, die es gestattet, dass der Randbereich 53 der Düsenplatte 51 mit der Düsenkanalwandung 31 in der beschriebenen Art und Weise in feste Verbindung geht. Der Trägerrahmen 56 gibt der Düsenplattenanordnung 50 als Ganzes eine höhere Eigenstabilität und vermindert darüber hinaus die Gefahr, dass durch das Montagewerkzeug 200 während der Montage eine Verletzung der Düsenplatte 51 eintritt.

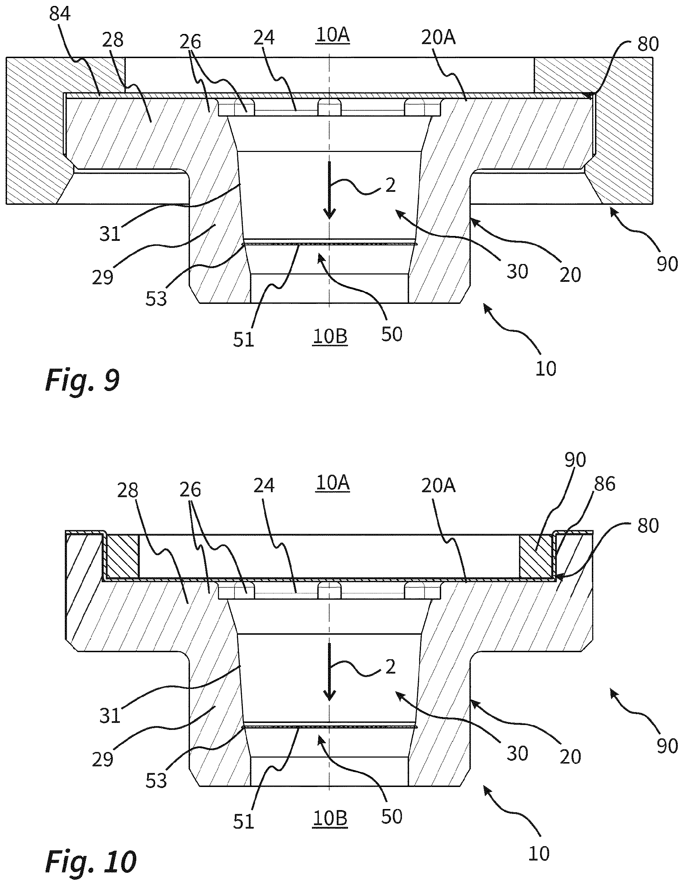

Fig. 9 zeigt eine alternative Gestaltung, bei der ein Klemmelement 90 in Form eines Klemmrings 90 vorgesehen ist, welches vorliegend statt der Schweißstelle 92 vorgesehen ist. Hier wird dementsprechend der Filter 80 nicht stoffschlüssig mit dem Kunststoffträger 20 verbunden, sondern durch den den Krempenabschnitt 28 umgreifenden Klemmring 90 in einem randseitigen Klemmbereich 84 gegen die Stirnfläche 20A des Kunststoffträgers axial gepresst.

Auch bei der Gestaltung der Fig. 10 wird der flache Filter 80 durch ein Klemmelement 90 in Form eines Klemmrings 90 fixiert. Hier allerdings wird der Klemmring 90 in eine Vertiefung des Kunststoffträgers 20 eingeschoben und klemmt zwischen seiner Außenseite und dem Rand der Vertiefung des Kunststoffträgers 20 das Material des Filters 80 in einem randseitigen Klemmbereich 86 radial ein.

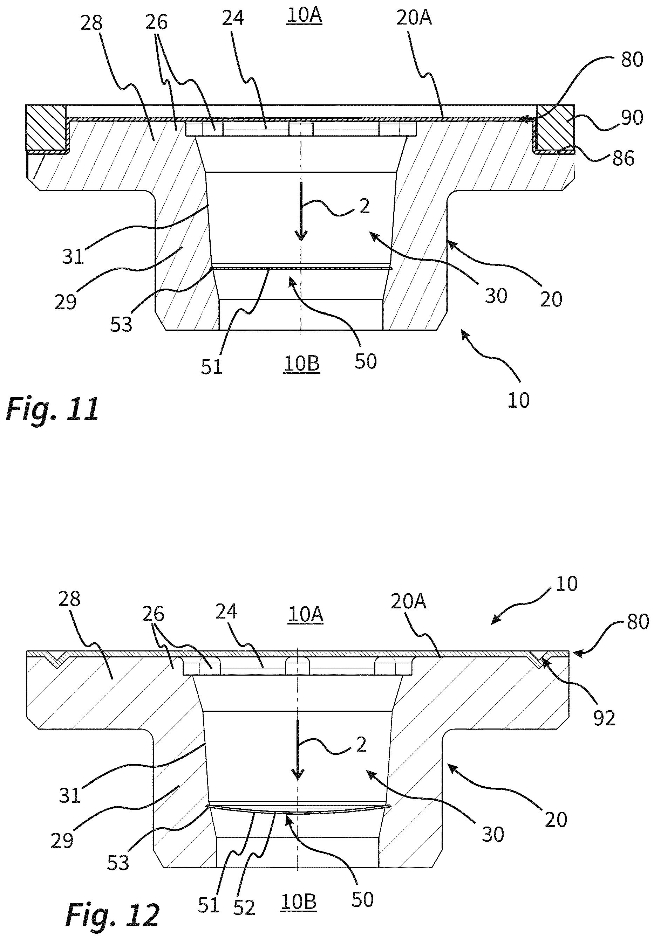

Die hierzu alternative Gestaltung der Fig. 11 zeigt das gleiche Grundprinzip mit radialer Einklemmung des Filters 80 im Klemmbereichen 86, wobei hier der Klemmring 90 außenseitig des Kunststoffträgers 20 angeordnet ist, so dass es eine Innenfläche des Klemmrings 90 ist, die in diesem Falle den Randbereich des Filters 80 einklemmt.

Fig. 12 zeigt eine alternative Gestaltung zu jener der Fig. 4, die sich von dieser dadurch unterscheidet, dass die Düsenplatte 51 eine gewölbte Konfiguration einnimmt. Eine solche gewölbte Konfiguration kann aufgrund der divergierenden Ausrichtung der Düsenöffnungen 52 von Vorteil sein und bewirkt einen weiter aufgefächerten Sprühstrahl.

Zur Herstellung einer solchen Gestaltung ist es zum einen möglich, die Düsenplatten 51 bereits vor Einbringung in den Düsenkanal 30 plastisch in eine gewölbte Formgebung zu drücken und das Verfahren gemäß den Fig. 5A bis 5E ansonsten unverändert durchzuführen.

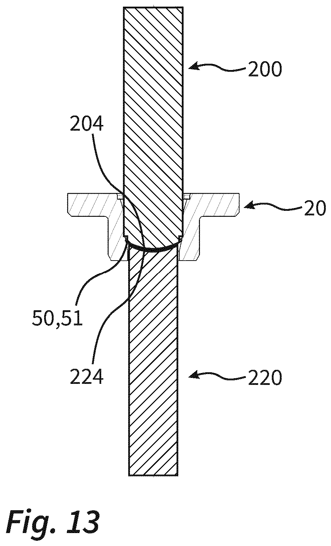

Wie Fig. 13 zeigt, kann alternativ jedoch auch vorgesehen sein, dass das Montagewerkzeug 200 eine konvex gewölbte Stirnfläche 204 aufweist und ein Hilfswerkzeug 220 von der gegenüberliegenden Seite in den Düsenkanal 30 eingeschoben wird. Dieses Hilfswerkzeug 220 weist eine ebenfalls gewölbte, jedoch diesmal konkav gewölbte Stirnfläche 224 auf. Gemeinsam drücken das Montagewerkzeug 200 und das Hilfswerkzeug 220 während der Montage die ursprünglich ebene Düsenplatte 51 elastisch in eine gewölbte Form und bringen sie in diesem elastisch verformten Zustand bis in ihre Endlage. Wenn dann das Montagwerkzeug und das Hilfswerkzeug 220 in entgegengesetzten Richtungen aus dem Düsenkanal 30 herausgezogen werden, so bleibt diese gewölbte Formgebung zumindest teilweise erhalten.

## Patentansprüche

1. Verfahren zur Herstellung einer Düseneinheit (10) für einen Flüssigkeitsspender (100) mit den folgenden Merkmalen:
a. das Verfahren dient der Herstellung einer Düseneinheit (10) mit einem Kunststoffträger (20), der von einer Eingangsseite (10A) bis zu einer Ausgangsseite (10B) von einem Düsenkanal (30) durchdrungen ist, sowie mit einer Düsenplattenanordnung (50), die eine Vielzahl von Düsenöffnungen (52) aufweist und in diesen Düsenkanal (30) eingesetzt wird, und
b. das Verfahren erfolgt unter Nutzung eines Montagewerkzeugs (200),
c. das Montagewerkzeug (200) weist eine Außenkontur (202) mit Übermaß gegenüber dem Düsenkanal (30) auf, so dass das Montagewerkzeug (200) durch Einführung in den Düsenkanal (30) und dortige Kraftbeaufschlagung einer Düsenkanalwandung (31) den Düsenkanal (30) elastisch aufweiten kann, und
d. das Montagewerkzeug (200) weist eine Stirnfläche (204) auf, die größer als eine Außenkontur (54) der Düsenplattenanordnung (50) ist, und
e. das Verfahren umfasst die folgenden Verfahrensschritte:
- der Kunststoffträger (20) wird in eine definierte Montagelage gebracht, und
- die Düsenplattenanordnung (50) sowie anschließend das Montagewerkzeug (200) werden von der Eingangsseite (10A) aus in einer Fügerichtung (2) in den Düsenkanal (30) eingeführt, und
- mit fortschreitendem Einfahren des Montagewerkzeugs (200) in den Düsenkanal (30) in Fügerichtung (2) wird der Düsenkanal (30) vom Montagewerkzeug (200) partiell elastisch aufgeweitet, so dass die Düsenplattenanordnung (50) bis in eine elastisch aufgeweitete Endlage gelangt, und
- das Montagewerkzeug (200) wird entgegen der Fügerichtung (2) aus dem Düsenkanal (30) herausgezogen, wobei die Düsenplattenanordnung (50) in der Endlage verbleibt und nach vollständiger Entnahme des Montagewerkzeugs (200) durch die elastische Rückstellung des Düsenkanals (30) durch die Düsenkanalwandung (31) gehalten ist.

2. Verfahren nach Anspruch 1 mit den folgenden weiteren Merkmalen:
a. die Düsenplattenanordnung (50) ist als einstückige Düsenplatte (51) ausgebildet, die mittels eines Stanzvorgangs aus einer Trägerplatte (150) mit einer Mehrzahl von Düsenplattenbereichen (51') ausgestanzt wird, und
b. nach dem Ausstanzen der Düsenplatte (51) wird die Düsenplatte (51) unmittelbar von der Eingangsseite (10A) aus in einer Fügerichtung (2) in den Düsenkanal (30) eingeführt.

3. Verfahren nach Anspruch 1 oder 2 mit den folgenden weiteren Merkmalen:
a. der Düsenkanal (30) weist mindestens einen konischen Teilabschnitt (36) auf, der an seinem in Richtung der Eingangsseite (10A) weisenden Ende einen Querschnitt (40) aufweist, der größer als die Außenkontur (202) des Montagewerkzeugs (200) ist, und der an seinem in Richtung der Ausgangsseite (10B) weisenden Ende einen Querschnitt aufweist, der kleiner als die Außenkontur (202) des Montagewerkzeugs (200) ist, und
b. beim Einführung des Montagewerkzeugs (200) in den Düsenkanal (30) gelangt das Montagewerkzeug (200) im konischen Teilabschnitt (36) in Kontakt mit der Düsenkanalwandung (31) des Düsenkanals (30).

4. Verfahren nach einem der vorstehenden Ansprüche mit dem folgenden weiteren Merkmal:
a. während des fortschreitenden Einfahrens des Montagewerkzeugs (200) in den Düsenkanal (30) in Fügerichtung (2) ist von der Ausgangseite (10B) aus ein Hilfswerkzeug (220) fluchtend mit dem Montagewerkzeug (200) in den Düsenkanal (30) eingeschoben.

5. Verfahren nach einem der vorstehenden Ansprüche mit den folgenden weiteren Merkmalen:
a. die Stirnfläche (204) des Montagewerkzeugs (200) weist eine konvex gewölbte Formgebung auf und/oder eine Stirnfläche (224) des Hilfswerkzeugs (220) weist eine konkav gewölbte Stirnfläche auf, und
b. während des Einfahrens des Montagewerkzeugs (200) in den Düsenkanal (30) drückt die konkave Formgebung und/oder die konvexe Formgebung die Düsenplattenanordnung elastisch und/oder plastisch in eine gewölbte Form.

6. Verfahren nach einem der vorstehenden Ansprüche mit mindestens einem der folgenden weiteren Merkmale:
a. der Kunststoffträger (20) in der definierten Montagelage derart ausgerichtet, dass die Eingangsseite (10A) nach oben weist und die Ausgangsseite (10B) nach unten weist, und/oder
b. nach Entfernen des Montagewerkzeugs (200) wird die Düsenplattenanordnung (50) zumindest abschnittsweise auf mindestens bis auf 100°C erwärmt.

## Claims

1. Method for producing a nozzle unit (10) for a liquid dispenser (100), having the following features:
a. the method is used to produce a nozzle unit (10) with a plastic carrier (20), which is traversed by a nozzle channel (30) from an inlet side (10A) to an outlet side (10B), and with a nozzle plate arrangement (50), which has a multiplicity of nozzle openings (52) and is inserted into this nozzle channel (30), and
b. the method is carried out using an assembly tool (200),
c. the assembly tool (200) has an outer contour (202) with an oversize in relation to the nozzle channel (30), such that the assembly tool (200), by being inserted into the nozzle channel (30) and by applying force there to a nozzle channel wall (31), can elastically expand the nozzle channel (30), and
d. the assembly tool (200) has an end face (204) which is larger than an outer contour (54) of the nozzle plate arrangement (50), and
e. the method comprises the following method steps:
- the plastic carrier (20) is brought into a defined assembly position, and
- the nozzle plate arrangement (50) and then the assembly tool (200) are inserted in a joining direction (2) into the nozzle channel (30) from the inlet side (10A), and
- with progressive insertion of the assembly tool (200) into the nozzle channel (30) in the joining direction (2), the nozzle channel (30) is partially expanded elastically by the assembly tool (200), such that the nozzle plate arrangement (50) is brought to an elastically expanded end position, and
- the assembly tool (200) is withdrawn from the nozzle channel (30) counter to the joining direction (2), wherein the nozzle plate arrangement (50) remains in the end position and, after complete removal of the assembly tool (200), is held by the nozzle channel wall (31) as a result of the elastic resetting of the nozzle channel (30).

2. Method according to Claim 1, having the following further features:
a. the nozzle plate arrangement (50) is designed as a one-piece nozzle plate (51) which, by means of a punching process, is punched out from a carrier plate (150) with a plurality of nozzle plate regions (51'), and
b. after the nozzle plate (51) has been punched out, the nozzle plate (51) is directly inserted into the nozzle channel (30) in a joining direction (2) from the inlet side (10A).

3. Method according to Claim 1 or 2, having the following further features:
a. the nozzle channel (30) has at least one conical sub-portion (36) which, at its end pointing in the direction of the inlet side (10A), has a cross section (40) which is larger than the outer contour (202) of the assembly tool (200), and, at its end pointing in the direction of the outlet side (10B), has a cross section which is smaller than the outer contour (202) of the assembly tool (200), and
b. during the insertion of the assembly tool (200) into the nozzle channel (30), the assembly tool (200) comes into contact with the nozzle channel wall (31) of the nozzle channel (30) in the conical sub-portion (36).

4. Method according to one of the preceding claims, having the following further feature:
a. as the assembly tool (200) is progressively inserted into the nozzle channel (30) in the joining direction (2), an auxiliary tool (220) is pushed, in alignment with the assembly tool (200), into the nozzle channel (30) from the outlet side (10B).

5. Method according to one of the preceding claims, having the following further features:
a. the end face (204) of the assembly tool (200) has a convexly curved shape and/or an end face (224) of the auxiliary tool (220) has a concavely curved end face, and
b. during the insertion of the assembly tool (200) into the nozzle channel (30), the concave shape and/or the convex shape presses the nozzle plate arrangement elastically and/or plastically into a curved shape.

6. Method according to one of the preceding claims, having at least one of the following further features:
a. the plastic carrier (20) is oriented, in the defined assembly position, in such a way that the inlet side (10A) points upwards and the outlet side (10B) points downwards, and/or
b. after removal of the assembly tool (200), the nozzle plate arrangement (50) is heated, at least in parts, to at least up to 100°C.

## Revendications

1. Procédé de fabrication d'une unité de buse (10) pour un distributeur de liquide (100) avec les caractéristiques suivantes :
a. le procédé sert à fabriquer une unité de buse (10) avec un support en matière plastique (20), qui est traversé par un canal (30) de buse depuis un côté d'entrée (10A) jusqu'à un coté de sortie (10B), ainsi qu'avec un ensemble (50) de plaques de buse, qui comporte une pluralité d'ouvertures (52) de buse et est inséré dans ledit canal (30) de buse, et
b. le procédé est effectué en utilisant un outil de montage (200),
c. l'outil de montage (200) comporte un contour extérieur (202) avec une surdimension par rapport au canal (30) de buse, de telle sorte que l'outil de montage (200) peut élargir élastiquement le canal (30) de buse par introduction dans le canal (30) de buse et par exposition à cet endroit d'une paroi (31) de canal de buse à l'action d'une force, et
d. l'outil de montage (200) comporte une surface frontale (204), qui est plus grande qu'un contour extérieur (54) de l'ensemble (50) de plaques de buse, et
e. le procédé comprend les étapes de procédé suivantes :
- le support en matière plastique (20) est amené dans une position de montage définie, et
- l'ensemble (50) de plaques de buse puis l'outil de montage (200) sont introduits dans le canal (30) de buse depuis le côté d'entrée (10A) dans une direction d'assemblage (2), et
- au fur et à mesure que l'insertion de l'outil de montage (200) dans le canal (30) de buse dans la direction d'assemblage (2) progresse, le canal (30) de buse est élargi en partie élastiquement par l'outil de montage (200) de telle sorte que l'ensemble (50) de plaques de buse parvient jusque dans une position finale élargie élastiquement, et
- l'outil de montage (200) est retiré du canal (30) de buse dans le sens opposé à la direction d'assemblage (2), l'ensemble (50) de plaques de buse restant dans la position finale et étant maintenu par la paroi (31) de canal de buse par le repositionnement élastique du canal (30) de buse après le retrait complet de l'outil de montage (200).

2. Procédé selon la revendication 1 avec les caractéristiques supplémentaires suivantes :
a. l'ensemble (50) de plaques de buse est réalisé comme une plaque de buse (51) d'un seul tenant, qui est estampée au moyen d'une opération d'estampage à partir d'une plaque de support (150) avec une multitude de zones (51') de plaques de buse, et
b. après l'estampage de la plaque de buse (51), la plaque de buse (51) est introduite dans le canal (30) de bus directement depuis le côté d'entrée (10A) dans une direction d'assemblage (2).

3. Procédé selon la revendication 1 ou 2, avec les caractéristiques supplémentaires suivantes :
a. le canal (30) de buse comporte au moins une section partielle (36) conique, qui comporte, sur son extrémité pointant en direction du côté d'entrée (10A), une section transversale (40), qui est plus grande que le contour extérieur (202) de l'outil de montage (200), et qui comporte, sur son extrémité pointant en direction du côté de sortie (10B), une section transversale qui est plus petite que le contour extérieur (202) de l'outil de montage (200), et
b. lors de l'introduction de l'outil de montage (200) dans le canal (30) de buse, l'outil de montage (200) parvient dans la section partielle conique (36) en contact avec la paroi (31) de canal de buse du canal (30) de buse.

4. Procédé selon l'une des revendications précédentes, avec la caractéristique supplémentaire suivante :
a. tandis que l'insertion de l'outil de montage (200) dans le canal (30) de buse dans la direction d'assemblage (2) progresse, un outil auxiliaire (220) est enfilé dans le canal (30) de buse dans l'alignement avec l'outil de montage (200) depuis le côté de sortie (10B).

5. Procédé selon l'une des revendications précédentes, avec les caractéristiques supplémentaires suivantes :
a. la face frontale (204) de l'outil de montage (200) présente une mise en forme à bombement convexe et/ou une face frontale (224) de l'outil auxiliaire (220) comporte une face frontale à bombement concave, et
b. pendant l'insertion de l'outil de montage (200) dans le canal (30) de buse, la mise en forme concave et/ou la mise en forme convexe poussent l'ensemble de plaques de buse élastiquement et/ou plastiquement en une forme bombée.

6. Procédé selon l'une des revendications précédentes, avec au moins une des caractéristiques supplémentaires suivantes :
a. le support en matière plastique (20) est orienté dans la position de montage définie de telle manière que le côté d'entrée (10A) pointe vers le haut et le côté de sortie (10B) pointe vers le bas, et/ou
b. après le retrait de l'outil de montage (200), l'ensemble (50) de plaques de buse est chauffé au moins par endroits à au moins 100 °C.
